# EUROPEAN PATENT APPLICATION

(11) **EP 4 047 008 A2**
(43) Date of publication of application: **24.08.2022**
(21) Application number: 20877215.2
(22) Date of filing: 08.10.2020
(51) Int. Cl.: C07K 7/06, C07K 5/09, A61K 38/00, A61P 17/14, A61K 8/64, A61Q 7/00

(54) **PEPTIDE FOR REDUCING HAIR LOSS AND PROMOTING HAIR GROWTH, AND COSMETIC COMPOSITION AND PHARMACEUTICAL COMPOSITION COMPRISING SAME**

(30) Priority: 18.10.2019 KR 20190129817
(71) Applicant: Incospharm Corporation, Daejeon 34036 (KR)
(72) Inventor: CHUNG, Hwa-Jee, Daejeon 34074 (KR); SHIN, Kayoung, Daejeon 35210 (KR); KIM, Heungjae, Daejeon 34010 (KR); PARK, Keedon, Daejeon 34889 (KR)
(74) Representative: Ladendorf, Oliver
(86) International application number: PCT/KR2020/013726
(87) International publication number: WO 2021/075791

(57) **Abstract**

The present invention relates to a peptide for reducing hair loss or promoting hair growth, and a cosmetic composition and a pharmaceutical composition comprising the same, and specifically, a cosmetic composition, and a pharmaceutical composition for treatment may be provided, which are capable of reducing hair loss and promoting hair growth by activating a Wnt/β-catenin signaling pathway, and at the same time, promoting the activity of the collagenase MMP-2 and ALP proteins and growth hormones needed for hair growth.

<110>    Incospharm Corporation

<120>    Peptides for alleviating hair loss and promoting hair growth,
         cosmetic composition and pharmaceutical composition containing
         the same

<130>    19P190933

<140>    10-2019-0129817
<141>    2019-10-18

<160>    2

<170>    KoPatentIn 3.0


<210>    1
<211>    5
<212>    PRT
<213>    Artificial Sequence

<220>
<223>    Dikkopf-1


<220>
<221>    HELIX
<222>    (244)..(248)

<400>    5
Ser Cys Arg Ile Gln
  1               5




<210>    2
<211>    3
<212>    PRT
<213>    Artificial Sequence

<220>
<223>    Dikkopf-1


<400>    10
Arg Ile Pro
  1

## Description

### [Technical Field]

The present invention relates to a peptide for preventing or improving alopecia and a composition comprising the same, and more particularly, to a peptide for reducing hair loss or promoting hair growth by activating a Wnt/β-catenin signaling pathway, and a cosmetic composition comprising the same.

### [Background Art]

Generally, people lose about 100 hairs a day on average, and new hairs grow at the same time, so the number of scalp hairs is not easily reduced. Hair loss refers to a condition in which there is no or poor hair in the area where there should be hair, and is also classified as a progressive disease caused by an insufficient supply of nutrients and poor active metabolism, which are necessary for hair generation.

Although the factors of hair loss are not yet clear, aging, genetic factors, stress, the action of male hormones, blood circulation disorders, abnormal sebum secretion, *Demodex folliculorum,* malnutrition, disruption in the immune system, skin diseases, etc. may cause hair loss in combination. As hair loss not only for men but also for women is increasing, external changes due to hair loss cause a lot of stress, psychological anxiety, and external complexes, and thus hair loss is a serious psychological problem for modern people.

There are various methods for treating hair loss, such as drug treatments, hair follicle transplantation surgery, folk remedies, oriental medicine treatment, and functional hair loss shampoo. Rogaine, the first safe hair loss treatment agent approved by the US Food and Drug Administration (FDA), promotes scalp blood circulation, but has no fundamental therapeutic effect. Pantogar supplies various amino acids and minerals contained in brewer's yeast to the scalp, but has no significant therapeutic effect. Drogen, that contains medicinal licorice ingredients of Donguibogam and Alimemazine tartrate as the main ingredient, is an oriental herbal hair growth treatment agent with insufficient evidence for hair growth. The above-mentioned hair loss treatment agents are not only accompanied by side effects but also are not fundamental treatments for hair loss. Currently, the most commonly used drugs for the treatment of hair loss approved by the US FDA are 2,4-diamino-6-piperidinopyrimidine-3-oxide (also referred to as 'Minoxidil (MINX)' preparation) and Propecia (trade name of Merck & Co., Inc.) whose main ingredient is finasteride, a specific inhibitor of type II 5α reductase. Minoxidil is a drug that induces hair growth by stimulating blood flow through the vasodilatory effect and supplying nutrients to the hair follicle, and is particularly known as having a good effect for reducing hair loss in hair whorl area. However, there is a disadvantage that it has to be used regularly for a long time, and it does not exert a very good effect on hair loss other than the hair whorl area. Propecia, which is administered orally, should also be taken continuously and regularly, and has been reported to have problems such as a high probability of birth defects if women take it for a long time, and side effects such as decreased libido and erectile dysfunction in some patients. Therefore, the development of a compound that may overcome the side effects of the minoxidil or finasteride and is excellent for preventing hair loss and promoting hair growth has been required.

The hair growth cycle consists of sequentially repeated three phases: a growth phase (Anagen), a transition phase (Catagen), and a resting or stationary phase (Telogen), and thereby the process wherein the hair grows and falls out is repeated. When there are more hair follicle cells that remain at the resting phase, hair begins to fall out and it becomes alopecia (baldness). Thus, treatment of fundamental hair loss requires approach to increasing the number of hair follicle cells in the anagen phase and reducing the number of cells in the telogen phase.

In order to solve the above problems, the inventors of the present invention synthesized a peptide fragment focusing on the association between the Wnt/β-catenin signaling system and hair growth and hair follicle stem cell, confirmed that there is an effect of reducing hair loss and promoting hair growth, thereby completing the present invention.

### [Related Art Document]

Kwack et al. Journal of Investigative Dermatology, 2012, 132, 6, 1554-1560

Jeong et al. Annals of Dermatology, 2017, 29, 1, 102-105

### [Disclosure]

### [Technical Problem]

An object of the present invention is to provide a peptide fragment involved in the Wnt/β-catenin signaling system for preventing, improving, or treating alopecia.

Another object of the present invention is to provide a cosmetic composition for improving alopecia and a pharmaceutical composition for preventing or treating alopecia, which are not limited to a specific region of the scalp, and are effective for hair loss symptoms throughout the scalp.

Still another object of the present invention is to provide a peptide that promotes hair growth by regulating the expression of proteins necessary for hair follicle cells.

Still further another object of the present invention is to provide a cosmetic composition and a pharmaceutical composition comprising a peptide that promotes hair growth as an effective ingredient.

### [Technical Solution]

In one general aspect, there is provided a peptide for preventing, improving, or treating alopecia comprising an amino acid sequence of SEQ ID NO: 5 or SEQ ID NO: 10:
SEQ ID NO: 5: SCRIQ, SEQ ID NO 10: RIP.

The peptide may activate Wnt/β-catenin signaling pathway.

The alopecia may be any one or more selected from the group consisting of alopecia areata, hereditary androgenetic alopecia, telogen alopecia, traumatic alopecia, genital alopecia, alopecia pityroides, seborrheic alopecia, and congenital alopecia.

In another general aspect, there is provided a cosmetic composition for improving alopecia, comprising a peptide comprising an amino acid sequence of SEQ ID NO: 5 or SEQ ID NO: 10 as an effective ingredient.

The effective ingredient may be included in an amount of 0.0001% to 10% by weight based on the total weight of the cosmetic composition.

In another general aspect, there is provided a pharmaceutical composition for preventing or treating alopecia, comprising a peptide comprising an amino acid sequence of SEQ ID NO: 5 or SEQ ID NO: 10, or a pharmaceutically acceptable salt thereof as an effective ingredient.

The effective ingredient may be included in an amount of 0.0001% to 10% by weight based on the total weight of the pharmaceutical composition.

The number of amino acid sequences of the peptide comprising SEQ ID NO: 5 may be 5 to 15, and the number of amino acid sequences of the peptide comprising SEQ ID NO: 10 may be 3 to 13.

In another general aspect, there is provided a polypeptide for preventing, improving, or treating alopecia, comprising the SEQ ID NO: 5 and SEQ ID NO: 10 with the number of amino acid sequences of 8 to 50.

In another general aspect, there are provided a peptide for promoting hair growth, consisting of 3 to 12 amino acid sequences comprising the amino acid sequence of SEQ ID NO: 5 or SEQ ID NO: 10, and a cosmetic composition for promoting hair growth comprising the same as an effective ingredient.

In another general aspect, there is provided a pharmaceutical composition for promoting hair growth, containing the peptide or a pharmaceutically acceptable salt thereof as an effective ingredient.

### [Advantageous Effects]

The peptide according to the present invention may selectively inhibit the binding of DKK-1, an inhibitor of the Wnt/β-catenin signaling pathway, to the LRP5/6 receptor, thereby activating the Wnt/β-catenin signaling pathway to stimulate growth hormones involved in the formation of new blood vessel, improvement of cell regeneration, enhancement of keratinocyte proliferation, and regulation of hair follicle growth in a desirable way, thereby providing the effect of preventing hair loss and promoting hair growth by maintaining the anagen phase of hair cycle.

In addition, the cosmetic composition and pharmaceutical composition containing the peptide as an active ingredient may be usefully used in the production of products for preventing, improving, or treating alopecia with few side effects.

### [Description of Drawings]

FIG. 1A is a western blot result of comparative analysis of changes in β-catenin and inactive/active phosphorylated GSK3β protein expression in dermal papilla cells by treatment with the peptide of SEQ ID NO: 5 according to Example 1 of the present invention, and FIG. 1B shows a graph for the results thereof.
FIG. 2A is a western blot result of comparative analysis of changes in β-catenin and inactive/active phosphorylated GSK3β protein expression in dermal papilla cells by treatment with the peptide of SEQ ID NO: 10 according to Example 1 of the present invention, and FIG. 2B shows a graph for the results thereof.
FIG. 3A is a result of screening a concentration having the highest β-catenin and inactive phosphorylated GSK3β protein expression activity by treating the peptide of SEQ ID NO: 5 at various concentrations according to Example 2 of the present invention.
FIG. 3B is a result of screening a concentration having the highest β-catenin and inactive phosphorylated GSK3β protein expression activity by treating the peptide of SEQ ID NO: 10 at various concentrations according to Example 2 of the present invention.
FIG. 4A is a result of confirming whether the activity of DKK-1 of SEQ ID NO: 5 and SEQ ID NO: 10 according to Example 3 of the present invention is inhibited.
FIG. 4B is a graph showing the degree of inhibition of the activity of DKK-1 of SEQ ID NO: 5 and SEQ ID NO: 10 according to Example 3 of the present invention based on the expression change rate of β-catenin.
FIGS. 5 and 6 are a result of analyzing the expression changes of the growth hormone proteins and genes that affect the maintenance of anagen phase and the inhibition of catagen progression of the hair cycle by the treatment of peptides of SEQ ID NO: 5 and SEQ ID NO: 10 according to Examples 4 to 6 of the present invention.
FIG. 7A is a result of analyzing the hair shaft elongation by treating human hair follicle organ with the peptide of SEQ ID NO: 5 according to Example 7 of the present invention.
FIG. 7B is a result of analyzing the hair shaft elongation by treating human hair follicle organ with the peptide of SEQ ID NO: 10 according to Example 7 of the present invention.

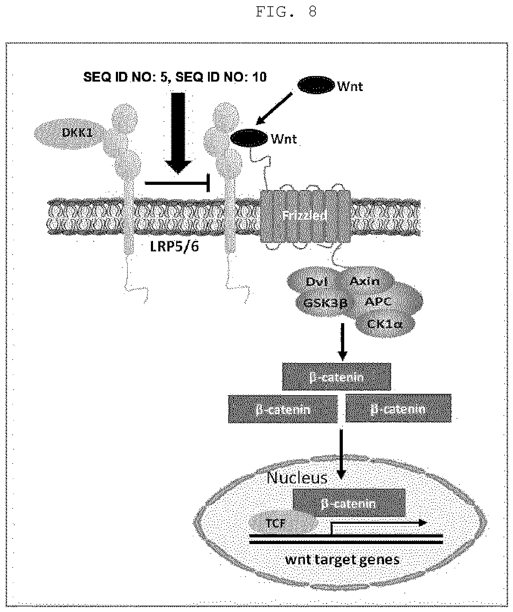

FIG. 8 shows a schematic diagram showing the mode of action of Wnt/β-catenin signaling pathway of the peptides of SEQ ID NO: 5 and SEQ ID NO: 10 according to the present invention.

### [Best Mode]

Hereinafter, the present invention will be described in detail. Unless otherwise defined, terms used in this specification should be interpreted as content commonly understood by those of ordinary skill in the art. The drawings and examples of the present specification are provided for those of ordinary skill in the art to easily understand and practice the present invention, and content that may obscure the gist of the present invention may be omitted from the drawings and examples, and the present invention is not limited to the drawings and examples.

The present invention provides a peptide for preventing, improving, or treating alopecia, comprising the amino acid sequence of SEQ ID NO: 5 or SEQ ID NO: 10.

[SEQ ID NO 5]: SCRIQ

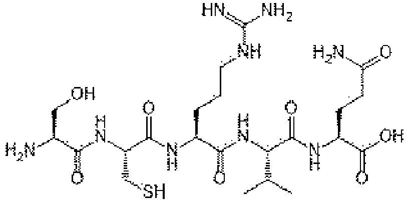

[SEQ ID NO 10]: RIP

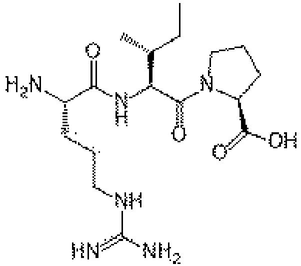

The peptide may activate Wnt/β-catenin signaling pathway.

Wnt is a cysteine-rich glycoprotein secreted by cells and regulates various developmental processes in all organisms by binding to receptors present on peripheral cells and regulating the expression of many genes through various signaling cascades.

Wnt/β-catenin signaling pathway plays an important role in an activation of hair growth and hair follicle stem cell. In the absence of Wnt ligand, β-catenin is phosphorylated by GSK3β (glycogen synthase kinase-3β), β-catenin degradation complex is formed in the cell, and β-catenin is degraded by the proteasome. However, when the Wnt ligand binds to the receptor LRP5 or LRP6, the activity of GSK3β is inhibited, thereby inhibiting the phosphorylation and degradation of β-catenin. Therefore, β-catenin accumulated in the cytoplasm moves to the nucleus to promote the expression of downstream target genes involved in the hair growth and differentiation.

The peptide comprising the amino acid sequence represented by SEQ ID NO: 5 or SEQ ID NO: 10 according to the present invention activates the Wnt/β-catenin signaling pathway by allowing the Wnt ligand to bind to the receptor LRP5 or LRP6, and consequently increases the physiological activity of hair follicle cells, which may lead to the effect of hair follicle regeneration.

More specifically, the peptide according to the present invention may compete with DKK-1 (Dickkopf-1) for binding to LRP5/6. DKK-1 is induced by dihydrotestosterone (DHT) as a potent antagonist of the Wnt/β-catenin signaling pathway. DHT is known as a hormone that directly causes hair loss, and binds to a specific part of hair follicle cells and promotes apoptosis and the transition of the hair cycle into the catagen phase. DKK-1 is a ligand with a very high affinity for LRP5/6, a receptor for Wnt ligand, and when DKK-1 binds selectively to LRP5/6, the formation of Wnt ligand-induced Frizzled-LRP5/6 complex is inhibited, and as the downstream signal, β-catenin is inactivated by phosphorylation , which may ultimately result in inhibition of hair growth.

The peptide according to the present invention may competitively bind to LRP5/6 with DKK-1, thereby inhibiting the interference of the Wnt/β-catenin signaling pathway by binding of DKK-1, and is rather possible to induce the expression of proteins associated with hair growth through activation of the Wnt/β-catenin signaling pathway.

The present invention provides a cosmetic composition for improving alopecia, containing the peptide as an active ingredient.

The effective ingredient may be included in an amount of 0.0001% to 10% by weight based on the total weight of the composition. Preferably, the effective ingredient may be included in an amount of 0.001% to 8% by weight, more preferably 0.01% to 3% by weight. Within the content range above, the peptide not only strengthens hair by activating growth factors and activating hair growth and hair follicles through improvement of blood circulation in the scalp, but also is effective in improving the scalp environment, and has a distinct effect of improving alopecia.

It is preferable to include 0.1 to 100 µM of the peptide comprising the amino acid sequence represented by SEQ ID NO: 5 or SEQ ID NO: 10 according to the present invention, and preferably 1 to 80 µM in the case of SEQ ID NO: 5, more preferably, 5 to 60 µM, 0.5 to 50 µM in the case of SEQ ID NO: 10, more preferably 10 to 50 µM may show better effect for improving alopecia and promoting hair growth.

The alopecia may be any one or more selected from the group consisting of alopecia areata, hereditary androgenetic alopecia, telogen alopecia, traumatic alopecia, genital alopecia, alopecia pityroides, seborrheic alopecia and congenital alopecia.

Specifically, the peptide may be used in the form of a salt, which is made by reacting an amino group with an appropriate acid, for example, as an organic acid addition salt, such as acetate, adipate, alginate, citrate, aspartate, benzoate, benzenesulfonate, bisulfate, butyrate, camphorate, camphorsulfonate, digluconate, glycerophosphate, hemisulfate, heptanoate, hexanoate, formate, fumarate, hydrochloride, hydrobromide, hydroiodide, 2-hydroxyethanesulfonate, lactate, maleate, mesitylenesulfonate, methanesulfonate, naphthylenesulfonate, nicotinate, 2-naphthalenesulfonate, oxalate, maleate, pamoate, pectinate, persulfate, 3-phenylpropionate, picrate, pivalate, propionate, succinate, tartrate, trichloroacetate, trifluoroacetate, phosphate, glutamate, bicarbonate, paratoluenesulfonate and undecanoate, but is not limited thereto.

In addition, examples of the acid that may be used to form the inorganic acid addition salt may include hydrochloric acid, hydrobromic acid, sulfuric acid, or phosphoric acid, but are not limited thereto.

The cosmetic composition may further include a buffer to maintain homeostasis of scalp cells. The buffer may be a complex buffer containing a monosaccharide, a polyalcohol, and an electrolyte compound.

The monosaccharide may be used without limitation as long as it can be used as a nutrient source in scalp cells. Specifically, as monosaccharides having 6 carbon atoms, any one or two or more selected from the group consisting of D-glucose, D-gulose, D-mannose, D-galactose, D-idose, D-talose, D-allose, D-altrose, D-fructose, D-tagatose, and L-sorbose may be used.

The monosaccharide may be included in an amount of 5% to 30% by weight, preferably 7% to 28% by weight, more preferably 10% to 25% by weight based on the buffer solution. When included in the above-mentioned content range, the feeling of use is excellent by forming an appropriate viscosity.

The electrolyte compound is generally used in a cosmetic composition to supply minerals deep within the scalp, and may be used without particular limitation as long as there is no allergic reaction and irritation in contact with the skin. Preferably, it may include any one or more selected from the group consisting of sodium chloride, potassium chloride, calcium chloride, and sodium sulfate.

In addition, the electrolyte compound may be included in an amount of 0.001% to 2% by weight, preferably 0.005% to 1% by weight, and more preferably 0.01% to 0.1% by weight based on the total cosmetic composition. When the electrolyte compound is included in the content range above, a healthy scalp environment may be created by maintaining the balance of acid and base inside and outside the cells and appropriately maintaining the osmotic pressure with the hair follicle cells.

The cosmetic composition of the present invention may be prepared in any formulation conventionally prepared in the art, which may have any one or more formulations selected from the group consisting of, for example, a solution, suspension, emulsion, paste, gel, cream, lotion, powder, soap, cleansing foam, oil, powder foundation, emulsion foundation, wax foundation, pack, massage cream, shampoo, rinse, treatment, and spray, but is not limited thereto.

When the formulation of the cosmetic composition for improving scalp health of the present invention is a paste, cream, or gel, the composition may further contain animal oil, vegetable oil, wax, paraffin, starch, tragacanth, cellulose derivative, polyethylene glycol, silicone, bentonite, silica, talc, or zinc oxide, and when the formulation of the cosmetic composition is a powder or spray, the cosmetic composition may further contain lactose, talc, silica, aluminum hydroxide, calcium silicate or polyamide powder, and in particular, when the formulation of the cosmetic composition is a spray, the cosmetic composition may further contain a propellant such as chlorofluorohydrocarbon, propane/butane or dimethyl ether.

When the formulation of the cosmetic composition of the present invention is a solution or emulsion, the cosmetic composition may further contain a solvent, solubilizer or emulsifier, which may be any one or more selected from the group consisting of water, ethanol, isopropanol, ethyl carbonate, ethyl acetate, benzyl alcohol, benzyl benzoate, propylene glycol, 1,3-butyl glycol oil, glycerol aliphatic ester, polyethylene glycol, and fatty acid ester of sorbitan.

When the formulation of the cosmetic composition of the present invention is a suspension, the cosmetic composition may further contain a liquid diluent such as water, ethanol or propylene glycol, ethoxylated isostearyl alcohol, suspending agent such as polyoxyethylene sorbitol ester, and polyoxyethylene sorbitan esters, microcrystalline cellulose, aluminum metahydroxide, bentonite, agar or tragacanth, etc.

In addition, the cosmetic composition may further contain any one or more appropriately selected as necessary from the group consisting of epidermal growth factor (EGF) that may prevent hair loss, transforming growth factor-a (TGFa), transforming growth factor-b (TGF-b), basic fibroblast growth factor-2 (bFGF), keratinocyte growth factor (KGF), stem cell factor (SCF), platelet-derived growth factor (PDGF), vascular endothelial growth factor (VEGF), and basic fibroblast growth factor (bFGF), and the peptide according to the present invention may provide the effect of creating and maintaining the scalp environment for promoting hair growth and hair generation by activating these growth factors.

The cosmetic composition according to the present invention is a part to which the cosmetic composition may be applied in general, and may be applied to any body part that requires hair generation as well as the scalp. For example, the cosmetic composition may be used to improve the condition of a part of hair or fur damaged by a scar due to trauma, or a wide forehead, M-shaped forehead, eyelashes, eyebrows, or atrichia pubis, which is desired for simple cosmetic effects.

The present invention may be a pharmaceutical composition for preventing or treating alopecia, containing the peptide or a pharmaceutically acceptable salt thereof as an effective ingredient.

The effective ingredient may be included in an amount of 0.0001% to 10% by weight based on the total weight of the composition as mentioned previously. In the content range above, the peptide not only strengthens hair by activating growth factors and activating hair growth and hair follicles through improvement of blood circulation in the scalp, but also is effective in improving the scalp environment and has a distinct effect of preventing or treating alopecia.

In addition, the number of amino acid sequences of the peptide comprising SEQ ID NO: 5 is 5 to 15, and the number of amino acid sequences of the peptide comprising SEQ ID NO: 10 may be 3 to 13. Specifically, for example, in the case of a peptide comprising SEQ ID NO: 5, it may be SCRIQ, EGLSCRIQ, EGLSCRIQK, EGLSCRIQKD, EGLSCRIQKDH, GLSCRIQKD, or GEGLSCRIQKDHH, but is not particularly limited thereto. In the case of a peptide comprising SEQ ID NO: 10, for example, if Q (glutamine) of the above-mentioned peptide is changed to P (proline), it may be RIP, SCRIP, EGLSCRIP, EGLSCRIPK, EGLSCRIPKD, EGLSCRIPKDH, GLSCRIPKD, or GEGLSCRIPKDHH, but is not particularly limited thereto.

The present invention provides a peptide composition for preventing, improving, or treating alopecia, comprising both SEQ ID NO: 5 and SEQ ID NO: 10. In the case of the peptide composition comprising both SEQ ID NO: 5 and SEQ ID NO: 10, it has the binding affinity which is greater in competitive binding with DKK-1 for LRP5/6, and may act more advantageously, and may exert the effect of inhibiting the binding of DKK-1 without inhibiting the binding of the Wnt ligand to the receptor.

In addition, the present invention provides a method for preventing or treating alopecia, comprising; administering a peptide composition comprising the amino acid sequence of SEQ ID NO: 5 and/or SEQ ID NO: 10.

In addition, the present invention provides a peptide for promoting hair growth, comprising the amino acid sequence of SEQ ID NO: 5 or SEQ ID NO: 10, a cosmetic composition for promoting hair growth containing the peptide as an effective ingredient, and a pharmaceutical composition containing the peptide or a pharmaceutically acceptable salt thereof.

The pharmaceutical composition may be administered orally, parenterally, intraarterially, intradermally, transdermally, intramuscularly, intraperitoneally, intravenously, subcutaneously or intranasally, but preferably parenterally, transdermally, or subcutaneously.

The composition of the present invention may further contain suitable carriers, excipients, and diluents conventionally used in the preparation of pharmaceutical composition.

Carriers, excipients and diluents that may be included in the composition comprising the effective ingredient of peptide of the present invention may be lactose, dextrose, sucrose, sorbitol, mannitol, xylitol, erythritol, maltitol, starch, acacia gum, alginate, gelatin, calcium phosphate, calcium silicate, cellulose, methyl cellulose, microcrystalline cellulose, polyvinyl pyrrolidone, water, methylhydroxybenzoate, propylhydroxybenzoate, talc, magnesium stearate, and mineral oil. In the case of formulation, it is prepared by using diluents or excipients such as fillers, extenders, binders, wetting agents, disintegrants, and surfactants that are usually used. A solid preparation for oral administration includes tablets, pills, powders, granules, capsules, etc., and such a solid preparation is prepared by mixing the extract with at least one excipient, for example, starch, calcium carbonate, sucrose or lactose, gelatin, etc. Also, in addition to simple excipients, lubricants such as magnesium stearate and talc may be used. A liquid preparation for oral administration may be suspensions, oral liquids, emulsions, syrups, etc., and include various excipients, for example, a wetting agent, a sweetener, an aromatic, a preservative, etc., in addition to water and liquid paraffin which are simple diluents commonly used.

When the pharmaceutical composition of the present invention is for parenteral administration, it may be any one or more formulations selected from the group consisting of creams, gels, patches, sprays, ointments, warnings, lotions, liniments, pastes, and cataplasmas during clinical administration, but is not limited thereto.

In order to formulate the pharmaceutical composition of the present invention into a formulation for parenteral administration, it is mixed in water together with a stabilizer or buffer to prepare a solution or suspension, which may be prepared in an ampoule or vial unit dosage form. The pharmaceutical composition may be sterilized or contain adjuvants such as preservatives, stabilizers, wetting agents or emulsification accelerators, salts and/or buffers for regulating osmotic pressure and other therapeutically useful substances, and may be formulated according to a normal method such as a mixing, granulation, or coating method.

Hereinafter, the present invention will be described with reference to the following examples. However, the following examples are for providing the present invention in detail, and the scope of the present invention is not limited to the following examples, and those of ordinary skill in the art may implement modified form.

### [Mode for Invention]

### [Preparation Example] Synthesis of peptides of SEQ ID NO: 5 and SEQ ID NO: 10

The peptides used in the present invention were synthesized by a solid phase method using Fmoc (9-fluorenylmethoxycarbonyl) as a protecting group of N**α** amino acid (Fmoc Solid Phase Peptide Synthesis), and the peptide was extended according to a method of HOBt-DIC (N-hydroxybenzotriazole-diisopropylcarbodiimide) (Wang C. Chan, Perter D. white, "Fmoc solid phase peptide synthesis" Oxford). The peptides of SEQ ID NO: 5 (serine-cysteine-arginine-isoleucine-glutamine, SCRIQ) and SEQ ID NO: 10 (arginine-isoleucine-proline, RIP) were synthesized by the above method, and after purification using high-performance liquid chromatography (Prep-HPLC, column C18, 10 **µ**m, 250 mm × 22 mm), SEQ ID NO: 5 (molecular weight measured by LC mass: 605.71) was obtained by lyophilization in a 69% yield of 83 mg, SEQ ID NO: 6 (molecular weight measured by LC mass: 600.69) was obtained in 72% yield of 86 mg, and SEQ ID NO: 10 (molecular weight measured by LC mass: 384.47) was obtained in 91% yield of 70 mg.

### [Example 1]. Confirmation of activation effect of Wnt/β-catenin signaling pathway of peptide

Western blot for β-catenin, GSK3β, and p-GSK3β protein expression was performed to analyze the activation of the intracellular Wnt/β-catenin signaling pathway by treatment with the peptides of SEQ ID NO: 5 and SEQ ID NO: 10 shown in Table 1 below.

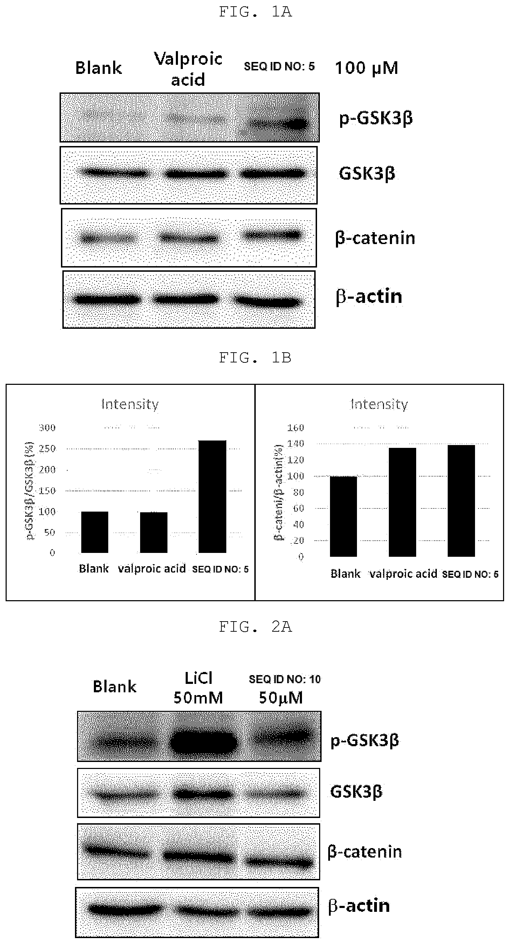

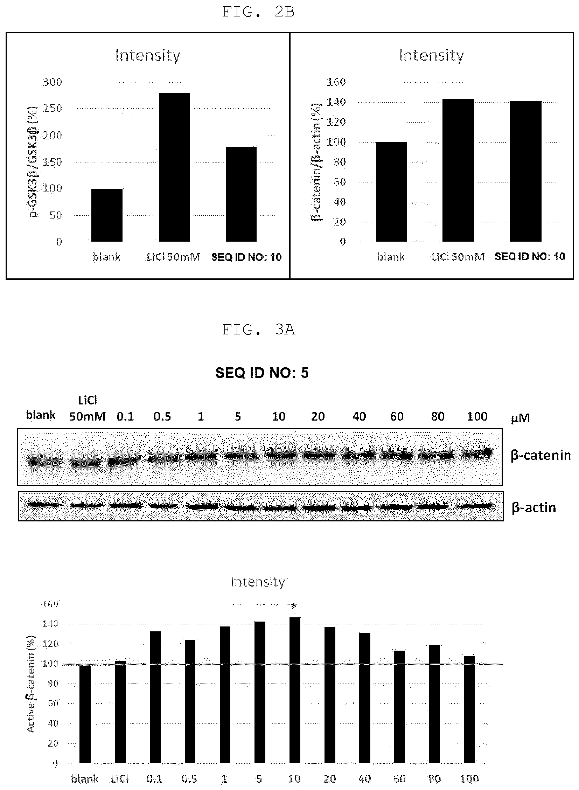

Human follicular dermal papilla cells (HFDPCs) were uniformly plated at the number of 1×10⁵ or 5×10⁴ cells in a 6-well plate and cultured in an incubator in DMEM (Dulbecco's Modified Eagle Media, Gibco BRL) for 24 hours at 37°C under 5% CO₂ conditions. Thereafter, the peptides of SEQ ID NO: 5 and SEQ ID NO: 10 were dissolved in water at a concentration of 10 mM to obtain a concentrate, which was diluted with a medium to a concentration of 200 uM, 100 uM, and 20 uM. After adding 1 ml of each dilution to each well containing 1 ml of the medium the cells were incubated for a certain period of time. After the culture was completed, the medium was removed, the cells were disrupted with SDS sample buffer. Each protein was separated by SDS-PAGE gel electrophoresis and transferred to a polyvinylidene fluoride (PVDF) membrane, and then non-specific binding was eliminated using a blocking buffer. The membrane was incubated with antibodies against active β-catenin, GSK3β, and p-GSK3β (Inactive) proteins and HRP-conjugated secondary antibody (anti-rabbit IgG HRP (sigma)), followed by an enhanced chemiluminescence (ECL) reaction using ECL prime kit (Amersham pharmacia), and ChemiDoc analysis, and the results are shown in FIGS. 1A, 1B, 2A, and 2B.

Through FIGS. 1A to 2B, when each of the peptides represented by SEQ ID NO: 5 and SEQ ID NO: 10 was treated, it can be seen that the expression of β-catenin and p-GSK3 β protein was significantly increased. It can be seen that the expression level is significantly increased compared to the control group not treated with the peptide.

**[Table 1]**

| SEQ ID NOs: 5 and 10 | | | |
|---|---|---|---|
| Name | Sequence | Name | Sequence |
| SEQ ID NO: 5 | SCRIQ | SEQ ID NO: 10 | RIP |

### [Example 2]. Screening for optimal concentration of Wnt/β-catenin signaling pathway activation

The optimal concentration for activating the Wnt/β-catenin signaling pathway was screened by treating with peptides of SEQ ID NO: 5 and SEQ ID NO: 10 according to the present invention by concentration. The specific experimental method is the same as in Example 1, except that in the case of SEQ ID NO: 5, the concentration was diluted to 0.1, 0.5, 1, 5, 10, 20, 40, 60, 80, 100 **µ**M, and the results are shown in FIG. 3A. The specific experimental method is the same as in Example 1, except that in the case of SEQ ID NO: 10, the concentration was diluted to 0.1, 0.5, 1, 5, 10, 20, 40, 50 **µ**M, and the results are shown in FIG. 3B.

As can be seen in FIGS.3A and 3B, it can be confirmed that both peptides of SEQ ID NO: 5 and SEQ ID NO: 10 according to the present invention increased the expression of active β-catenin protein at all concentrations from low concentration 0.1 **µ**M to high concentration 100 **µ**M or 50 **µ**M. In addition, it can be confirmed that there is an optimal concentration that most effectively increases the expression of active β-catenin protein of the peptides of SEQ ID NO: 5 and SEQ ID NO: 10, and through this, the effect does not increase in a concentration-dependent manner.

### [Example 3]. Confirmation of inhibition of DKK-1 activity

By treatment with the peptides of SEQ ID NO: 5 and SEQ ID NO: 10 according to the present invention, it was analyzed whether the Wnt/β-catenin signaling pathway inhibited by DKK-1 was reactivated.

Specifically, human follicular dermal papilla cells (HFDPCs) were uniformly plated at the number of 5×10⁴ cells in a 12-well plate and cultured in an incubator in DMEM (Dulbecco's Modified Eagle Media, Gibco BRL) for 24 hours at 37°C under 5% CO₂ conditions. After culturing, each well was treated with the recombinant protein DKK-1 to inhibit the Wnt/β-catenin signaling pathway, and further treated with the peptides of SEQ ID NO: 5 and SEQ ID NO: 10 of the present invention at appropriate concentrations, followed by culturing for a certain period of time.

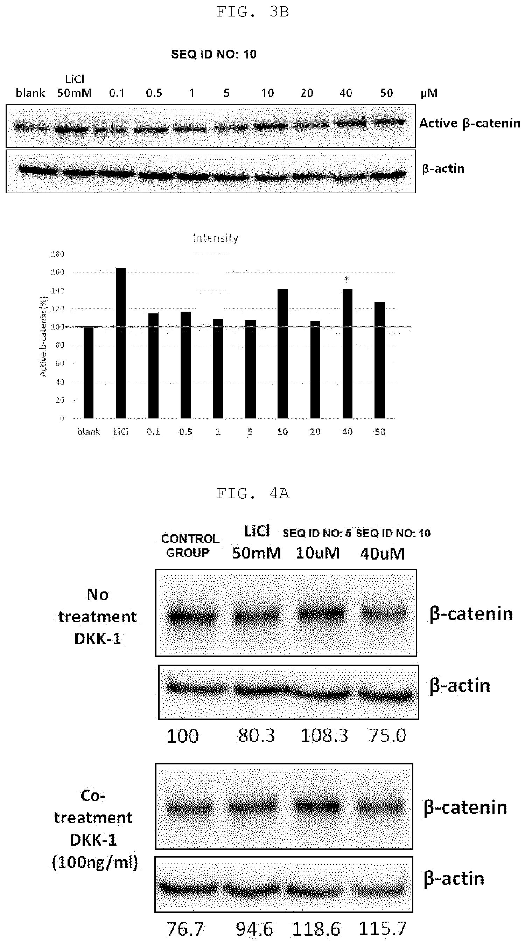

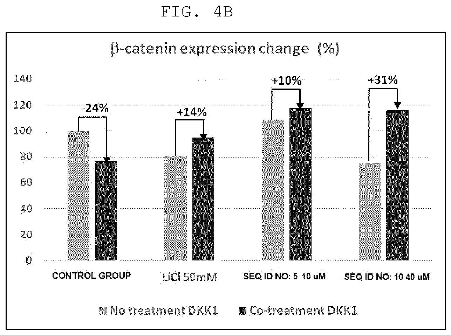

After the culture was completed, the medium was removed, the cells were disrupted with SDS sample buffer. Each protein was separated by SDS-PAGE gel electrophoresis and transferred to a PVDF membrane (polyvinylidene fluoride), and then non-specific binding was eliminated using a blocking buffer. The membrane was incubated with an antibody against β-catenin and p-GSK3β protein and an HRP-conjugated secondary antibody (anti-rabbit IgG HRP(sigma)), thereto, an enhanced chemiluminescence (ECL) reaction using ECL prime kit (Amersham pharmacia) and chemiDoc analysis were performed, and the results are shown in FIGS. 4A and 4B.

As can be seen from FIGS. 4A and 4B, it could be confirmed that in the case of control group, the expression level of non-phospho(active)β-catenin was decreased by about 24% when the recombinant protein DKK-1 was treated, indicating that the Wnt/β-catenin signaling pathway was inhibited by recombinant DKK-1. On the other hand, it could be confirmed that when the peptides of SEQ ID NO: 5 and SEQ ID NO: 10 of the present invention were simultaneously treated with recombinant DKK-1, the expression level of non-phospho (active) β-catenin was recovered by increasing about 10% to 31%, respectively. These results support the fact that the peptide according to the present invention may competitively bind to LRP5/6 with DKK-1 to inhibit the interference of the Wnt/β-catenin signaling pathway by binding of DKK-1, and may rather induce the expression of proteins associated with hair growth through activation of the Wnt/β-catenin signaling pathway.

### [Example 4]. Confirmation of increased expression of hair-related growth hormone

To confirm changes in proteins or genes of growth hormones, VEGF, IGF-I, FGF10, FGF1 and FGF7 that affect the hair cycle anagen phase by treating peptides of SEQ ID NO: 5 and SEQ ID NO: 10 according to the present invention, Western blot and RT-PCR were performed.

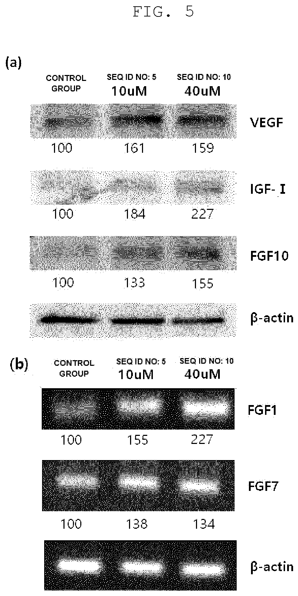

Specifically, human follicular dermal papilla cells (HFDPCs) were uniformly plated with western blot experimental method at the number of 1×10⁵ cells in a 6-well plate and cultured in an incubator in DMEM (Dulbecco's Modified Eagle Media, Gibco BRL) for 24 hours at 37°C under 5% CO₂ conditions. The peptides of SEQ ID NO: 5 and SEQ ID NO: 10 of the present invention were dissolved in water at a concentration of 10 mM to obtain a concentrate, which was diluted with a medium to an optimal concentration of 10 or 40 **µ**M, respectively. Thereafter, 1 ml of each dilution solution was added in each well containing 1 ml of medium, , and incubated for 48 hours. After the culture was completed, the medium was removed and the cells were disrupted with SDS sample buffer, followed by separating each protein on SDS-PAGE gel electrophoresis, transferring to a polyvinylidene fluoride (PVDF) membrane, and then removing non-specific binding using a blocking buffer. The membrane was incubated with antibodies against proteins of VEGF, IGF1, FGF2 and FGF10 and HRP-conjugated secondary antibody (anti-rabbit IgG HRP (sigma)), followed by an enhanced chemiluminescence (ECL) reaction using ECL prime kit (Amersham pharmacia), and ChemiDoc analysis, and the results are shown in FIG. 5.

In addition, as a specific RT-PCR test method, peptides of SEQ ID NO: 5 and SEQ ID NO: 10 of the present invention were applied with the optimal concentration of 10 or 40 **µ**M to human dermal papilla cells cultured in the same manner as above, and cultured for a certain period of time. After completion of the culture, the cells were disrupted with Trizol (Ambion), total mRNA was collected with chloroform/isopropanol, and cDNA was synthesized with reverse transcriptase. RT-PCR (Thermal Cycler, Bio-rad) was performed using primers specific for FGF1 and FGF7, and the amplified product was analyzed by agarose gel electrophoresis, and the results are shown in FIG. 5.

As can be seen from FIG. 5, it can be confirmed that the expression of proteins of VEGF, IGF-I and FGF10, genes of FGF1 and FGF7 proteins was increased by peptides of SEQ ID NO: 5 and SEQ ID NO: 10. These are proteins related to hair growth factors, and by increasing their expression, the effect of promoting hair growth of the peptides according to the present invention may be expected.

### [Example 5]. Confirmation of increase of expression MMP-2 (matrix metalloproteinase-2) and ALP (alkaline phosphatase)

RT-PCR was performed to confirm changes in the MMP-2 protein gene, a Type IV collagenase that affects the anagen phase of the hair cycle, and the ALP protein gene, a representative factor of the anagen phase of the hair cycle, by treating peptides of SEQ ID NO: 5 and SEQ ID NO: 10 according to the present invention.

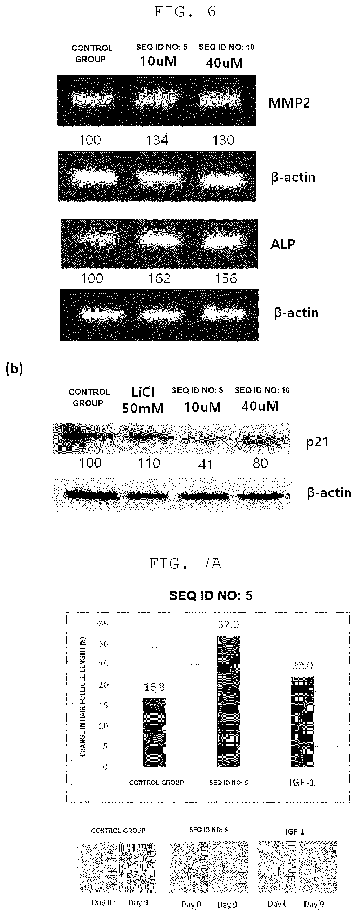

A specific RT-PCR test method was performed in the same manner as in Example 4. RT-PCR (Thermal Cycler, Bio-rad) was performed using primers specific for MMP-2 and ALP, and the amplified product was analyzed by agarose gel electrophoresis, and the results are shown in FIG. 6A.

As can be seen in FIG 6A, it can be confirmed that the gene expression of the MMP-2 and ALP protein is increased by peptides of SEQ ID NO: 5 and SEQ ID NO: 10 compared to the control group, and with an increase of their expression, the effect of promoting hair growth of the peptide according to the present invention may be expected.

### [Example 6]. Confirmation of inhibition of cell cycle-related protein p21

As one of the target proteins of the Wnt/β-catenin signaling pathway, p21 (cyclin-dependent kinase inhibitor 1a, Cdknla) is highly expressed during the catagen phase, and functions to inhibit entry into the anagen phase by inhibiting the expression of cyclin-dependent kinases (Cdks). In order to analyze whether p21 may be inhibited by treatment with peptides of SEQ ID NO: 5 and SEQ ID NO: 10, Western blot for an expression of p21 protein was performed.

The specific experimental method was performed in the same manner as in Example 4. After transferring to a PVDF membrane (polyvinylidene fluoride), non-specific binding was eliminated using a blocking buffer. The membrane was react with an antibody against p21 protein and HRP-conjugated secondary antibody (anti-mouse IgG HRP (sigma)), and followed by an enhanced chemiluminescence (ECL) reaction using an ECL prime kit (Amersham pharmacia), and ChemiDoc analysis, and the results are shown in FIG. 6B.

As can be seen in FIG. 6B, it can be confirmed that the p21 protein was significantly reduced compared to the control group by peptides of SEQ ID NO: 5 and SEQ ID NO: 10. Therefore, the effect of promoting hair growth by the peptides of SEQ ID NO: 5 and SEQ ID NO: 10 according to the present invention can be expected.

### [Example 7]. Confirmation of effect of promoting hair shaft growth in human hair follicles

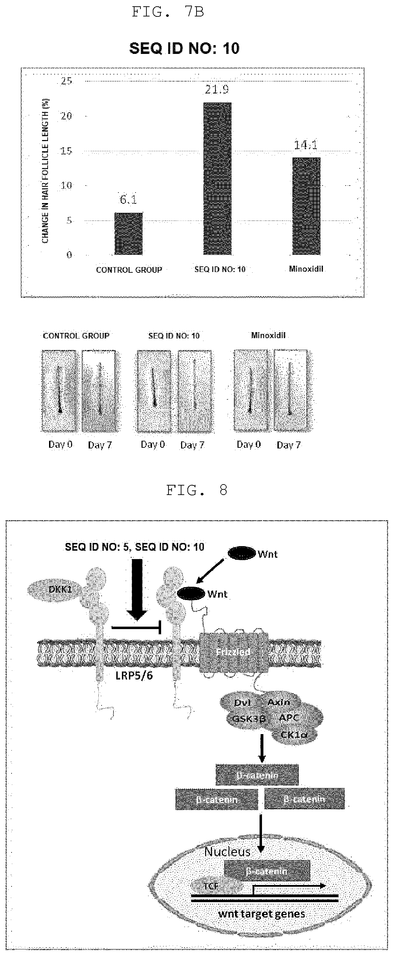

After separating the surrounding tissues, the hair follicles isolated from the human scalp tissues were stored in Earle's balanced salts solution (EBSS; SigmaAldrich). The hair follicles in anagen phase were carefully separated under a microscope and then used in the experiment. The isolated hair follicles were cultured in Williams medium E (Gibco, Grand Island, NY, USA) at 37°C, 5% CO₂ and 95% air with an addition of 2 mM L-glutamine (Gibco, NY, USA), 10 **µ**g/ml insulin (SigmaAldrich), 10 ng/ml hydrocortisone (SigmaAldrich), 100 Unit/ml penicillin and 100 **µ**g/ml streptomycin. The length of the hair shaft grown after culturing for 7 or 10 days by treating the compounds of SEQ ID NO: 5 (100 **µ**M) and SEQ ID NO: 10 (16.6 **µ**M) of the present invention was measured using Image J (version 1.52a NIH, Bethesda, MD, USA) and analyzed, and the results are shown in FIGS. 7A and 7B. At this time, as a positive control group, Minoxidil (10 **µ**M) or IGF-1 (10 ng/ml) was treated.

As can be seen in FIGS. 7A and 7B, the groups that treated with SEQ ID NO: 5 and SEQ ID NO: 10 of the present invention, respectively, showed a superior hair shaft growth compared to the IGF-1 or minoxidil positive control group.

### Free text of sequence listing

Information of sequences of SEQ ID NOs: 5 and 10 was submitted in a separate file form.

## Claims

1. A peptide for preventing, improving, or treating alopecia, comprising an amino acid sequence of SEQ ID NO: 5 or SEQ ID NO: 10:
SEQ ID NO: 5: SCRIQ
SEQ ID NO: 10: RIP.

2. The peptide of claim 1, wherein the peptide activates a Wnt/β-catenin signaling pathway.

3. The peptide of claim 1, wherein the alopecia is any one or more selected from the group consisting of alopecia areata, hereditary androgenetic alopecia, telogen alopecia, traumatic alopecia, genital alopecia, alopecia pityroides, seborrheic alopecia, and congenital alopecia.

4. A cosmetic composition for improving alopecia, comprising the peptide of claim 1 as an effective ingredient.

5. The cosmetic composition of claim 4, wherein the effective ingredient is included in an amount of 0.0001% to 10% by weight based on a total weight of the composition.

6. A pharmaceutical composition for preventing or treating alopecia, comprising the peptide of claim 1 or a pharmaceutically acceptable salt thereof as an effective ingredient.

7. The peptide of claim 1, wherein the number of amino acid sequences of peptide comprising the SEQ ID NO: 5 is 5 to 15, and the number of amino acid sequence of peptide consisting of the SEQ ID NO:10 is 3 to 13.

8. The peptide composition of claim 1, wherein the peptide comprises the peptide comprising the SEQ ID NO: 5 and the peptide comprising the SEQ ID NO: 10.

9. A peptide for promoting hair growth, comprising an amino acid sequence of SEQ ID NO: 5 or SEQ ID NO:10:
SEQ ID NO: 5: SCRIQ
SEQ ID NO: 10: RIP.

10. A cosmetic composition for promoting hair growth, comprising the peptide of claim 9 as an effective ingredient.

11. A pharmaceutical composition for promoting hair growth, comprising the peptide of claim 9 or a pharmaceutically acceptable salt thereof as an effective ingredient.
